# EUROPÄISCHE PATENTSCHRIFT

(11) **EP 0 576 740 B1**
(45) Veröffentlichungstag und Bekanntmachung des Hinweises auf die Patenterteilung: **29.07.1998**
(21) Anmeldenummer: 92250170.5
(22) Anmeldetag: 29.06.1992
(51) Int. Cl.: A61K 49/04, A61K 49/00

(54) **Verfahren und Vorrichtung zur Herstellung eines Kontrastmediums aus einem Konzentrat**
Process and device for the preparation of a contrast medium from a concentrate
Procédé et dispositif de préparation d'un milieu de contrast en portant d'un concentré

(43) Veröffentlichungstag der Anmeldung: 05.01.1994
(73) Patentinhaber: SCHERING AKTIENGESELLSCHAFT, 13353 Berlin (DE)
(72) Erfinder: Kämpfe, Michael, Dr., W-1000 Berlin 41 (DE); Better, Bernard, W-1000 Berlin 51 (DE)

(56) Entgegenhaltungen:
- FR-A- 2 545 001
- STN SERVER & FILE MEDLINE KARLSRUHE 'AN 88138677'

## Beschreibung

Die Erfindung betrifft ein verfahren zur Herstellung eines Kontrastmediums in einsetzbaren Dosierungsformen. Sie betrifft weiterhin eine Vorrichtung zur Herstellung eines verabreichungsfähigen Kontrastmediums aus wenigstens einem Konzentrat und wenigstens einem Verdünnungsmittel.

Kontrastmedien sind wichtige Hilfsmittel für diagnostische Verfahren, wie Ultraschall-, Röntgen- oder Kernresonanz-Diagnosen, geworden. So verbessert beispielsweise die Verabreichung eines Kontrastmittels an einen Patienten die Auswertung eines Röntgenbildes, da hierdurch kontrastreichere Bilder erhalten werden. Aufgrund dieser Tatsache kann das medizinische Personal die durch ein solches Bild zur Verfügung gestellten Informationen besser auswerten.

Was nun die Dosierung betrifft, sind zahlreiche Faktoren bei der Bestimmung der Dosierungsmenge und der Dosierungskonzentration des zu verabreichenden Kontrastmittels zu berücksichtigen, beispielsweise die Art der angewandten diagnostischen Prüfung, die Art und Weise, in der diese diagnostische Prüfung durchgeführt wird, der zu untersuchende Körperbereich des Patienten sowie das Alter, das Körpergewicht und der Gesundheitszustand des Patienten. Angesichts dieser zahlreichen Faktoren sind Kontrastmittel, wie Röntgenkontrastmittel, in einer Reihe von Konzentrationen erhältlich, beispielsweise 120-400 mg J/ml, und in einer Vielzahl von Dosierungsmengen, beispielsweise 1 ml - 250 ml. Trotz der zahlreichen Konzentrationen und Dosierungsmengen kann jedoch nicht jeder individuellen Situation des einzelnen Patienten Rechnung getragen werden.

Trotz dieser Vielfalt von Dosierungsformen, die im Handel erhältlich sind (Konzentrationen und Größen), ist es in der klinischen Anwendung häufig unvermeidbar, daß nur eine Teilmenge für die Untersuchung benötigt und die übrig bleibende Menge verworfen wird. Ein Wiedereinsatz ist dabei ausgeschlossen, da dies eine Kontraindikation darstellen würde.

Aufgrund der zahlreichen diagnostischen Untersuchungen und der hohen Preise der modernen Kontrastmedien stellt jedoch ein solcher Abfall einen beträchtlichen Kostenfaktor für die Klinik bzw. die medizinische Praxis dar. Desweiteren müssen zahlreiche Formen von Kontrastmedien im Lager gehalten werden, wodurch die Bereithaltungskosten erheblich steigen. In den Fällen, in denen eine neue Untersuchungstechnik neue Präparationsformen erfordert, stellen die häufig langen Zeitintervalle für die Entwicklung und Zulassung neuer Flaschengrößen und/oder -konzentrationen ebenfalls einen Nachteil dar.

Desgleichen stellt die Vielzahl von Darreichungsformen von Kontrastmedien auf der Herstellungsseite ebenfalls einen Nachteil dar, da nur relativ kleine Produktionschargen hergestellt werden können, der Lagerbedarf erhöht ist, die Logistik schwierig ist und teurere und verlängerte Entwicklungs- und Zulassungszeiten die Folge sind. Zusätzlich besteht nur eine geringe Flexibilität in der Kontrastmedienproduktion für Veränderungen der Präparationsformen, wie sie vom Markt gefordert werden.

Diese Nachteile sind bis heute nicht ausgeräumt.

Auf dem pharmazeutischen Gebiet sind eine Vielzahl von Abfüllvorrichtungen bekannt, mit denen portionsweise Lösungen gemischt werden können, beispielsweise aus der WO 84/00139 oder der DE-OS 33 15 031.

So beschreibt die WO 84/00139 eine Mischvorrichtung, die geeignete Mittel zum Überwachen der Abfüllung von pharmazeutischen Lösungen aufweist. Es werden dabei Lösungen abgefüllt, die verschiedenen Behältern entnommen werden. Eine gezielte Mischung zur Sicherstellung gleichmäßiger Konzentrationen in der fertigen, anwendungsgerechten Lösung findet jedoch nicht statt.

Auch mit der aus der DE-OS 33 15 031 bekannten Mischvorrichtung werden nur Infusionslösungen zur parenteralen Ernährung vermischt, ohne daß auf die Problematik der Herstellung von Kontrastmittellösungen Bezug genommen würde.

Im übrigen sind beide bekannten Vorrichtungen nicht zur Mischung von Flüssigkeiten mit stark unterschiedlicher Dichte geeignet, da ein einfaches Zusammenbringen solcher Flüssigkeiten nicht eine gleichmäßig verdünnte Lösung hervorbringt, wie sie für die genannten Anwendungen erforderlich ist.

Der Erfindung liegt daher die Aufgabe zugrunde, ein Verfahren der eingangs erwähnten Art zur Verfügung zu stellen, mit dem patientenspezifisch, indikationsspezifisch dosierungsgerechte Kontrastmittelzusammensetzungen hergestellt werden können, ohne daß eine Vielzahl von Darreichungsformen bereitgehalten werden muß und/oder erhebliche Abfallmengen auftreten.

Darüber hinaus können Kontrastmittel und gegebenenfalls gewünschte "Additive" in einer Applikation verabreicht werden, wodurch der Komfort für den Patienten erhöht wird.

Diese Aufgabe wird durch die kennzeichnenden Merkmale des Anspruchs 1 gelöst.

Bei dem erfindungsgemäßen Verfahren werden ein oder mehrere Kontrastmittelkonzentrate in entsprechenden Behältern eingesetzt, die fließfähig und hochkonzentriert sind. Diese Konzentrate werden in Form von Lösungen, Dispersionen oder als fließfähige (rieselfähige) Pulver eingesetzt und in entsprechenden Konzentratbehältern vorgelegt.

Desweiteren werden ein oder mehrere Verdünnungsmittel zur Vermischung mit den Konzentraten eingesetzt, beispielsweise steriles oder unsteriles Wasser. Andererseits können jedoch aber auch pharmazeutische Lösungen als Verdünnungs- und/oder Lösungs- oder Dispersionsmittel für die Verdünnung des Konzentrats eingesetzt werden. Schließlich können selbst verdünnte Kontrastmittellösungen zur Vermischung mit den Kontrastmittelkonzentraten eingesetzt werden.

Die Kontrastmittelkonzentrate können Konzentrationen aufweisen, die über denjenigen Werten liegen, die man bisher aus applikationstechnischen Gründen (z. B. Viskosität) hergestellt wurden. So werden beispielsweise Röntgenkontrastmittelkonzentrate mit einem Gehalt von 350-450 mg J/ml eingesetzt oder NMR-Kontrastmittel in Lösung oder Dispersion mit 0,5 mol/Liter in Form von Gadopentetat-Dimeglumine oder anderer auf paramagnetischen Ionen basierenden NMR-Kontrastmitteln. Es können jedoch auch verschiedene Kontrastmittelkonzentrate für verschiedene medizinische Techniken eingesetzt werden, beispielsweise für die Röntgen-, Kernresonanz- oder Ültraschalldiagnostik.

Sofern die Kontrastmittelkonzentrate in Pulverform vorliegen, können diese Pulver zu 100 % aus dem jeweiligen Kontrastmittel bestehen oder gegebenenfalls weitere feste galenische Hilfsstoffe (die z.B. die Rieselfähigkeit des Kontrastmittels verbessern) enthalten. Das Gewichtsverhältnis vom Kontrastmittel zum Hilfsstoff kann beliebig gewählt werden.

Röntgenkontrastmittel können beispielsweise enthalten Iotrolan, Iopromid, Iohexol, Iosimid, Metrizamid, Salze von Amidoessigsäure, Iotroxinsäure, Iopamidol, 5-Hydroxyacetamido-2,4,6-triiodo-isophthalsäure-(2,3-dihydroxy-N-methylpropyl)-(2-hydroxyethyl)-diamid, 3-Carbamoyl-5-[N-(2-hydroxyethyl)-acetamido]-2,4,6-triiodo-benzoesäure-[(1RS, 2SR)-2,3-dihydroxy-1-hydroxymethylpropyl]amid und Dispersionen von wenig löslichen Röntgenkontrastmitteln, wie Iodipamidethylester.

Zu NMR-Kontrastmittelmedien gehören beispielsweise Gadolinium DTPA, Gadolinium DOTA, der Gadoliniumkomplex von 10[1-Hydroxymethyl-2,3-dihydroxypropyl]-1,4,7-tris-[(carboxymethyl)-1,4,7,10-tetraazacyclodecan], Eisen- oder Manganporphyrinchelate und stabile Magnetitdispersionen.

Zu Ultraschallkontrastmedien gehören beispielsweise Dispersionen von Galaktosemikropartikeln mit oder ohne Additiven in Wasser oder eine Galaktoselösung und Dispersionen von Mikrokugeln von eingeschlossener Luft (beispielsweise Cyanacrylate oder Albuminmikrokugeln) sowie andere injizierbare Mikropartikel.

Gemäß dem erfindungsgemäßen Verfahren wird eine vorbestimmte Menge von Kontrastmittelkonzentrat mit einer vorbestimmten Menge von Verdünnungsmittel und gegebenenfalls sonstiger Additive vermischt, um die gewünschte Dosierungsform des Kontrastmittels zu formulieren.

Es können auch Kontrastmittel für verschiedene Anwendungsgebiete miteinander gemischt werden. Hierdurch können am Patienten mit nur einer Applikation zwei oder mehrere verschiedene Untersuchungen durchgeführt werden, was eine deutliche Verbesserung des Patientenkomforts darstellt. Eine solche Mischung von zwei oder mehreren Kontrastmitteln wird üblicherweise sofort nach dem Mischen verbraucht, so daß keine Stabilitätsprobleme auftreten.

Als Verdünnungsmittel kommt beispielsweise ein physiologisch verträgliches flüssiges Trägermittel in Frage, das gegebenenfalls einsetzbare Additive enthält. So kann beispielsweise als Verdünnungsmittel steriles oder unsteriles Wasser eingesetzt werden, das gegebenenfalls Puffersubstanzen und/oder isotonisch wirkende Additive sowie gegebenenfalls Komplexierungsmittel, wie CaNa₂EDTA, aufweist.

Als Verdünnungsmittel kann auch ein vergleichsweise niedriger konzentriertes Kontrastmittel eingesetzt werden, beispielsweise in einer Konzentration von 0-0,5 mol/l bzw. 0-350 mgI/l bzw. 0-100 mg Partikel/ml.

Einsetzbare Additive sind beispielsweise anorganische oder organische Salze oder Puffersubstanzen, wie Natriumchlorid, tris-Puffer, Phosphatpuffer, Citratpuffer, Glycinpuffer, Citrat-Phosphat-Puffer, Maleatpuffer und dergl. Weiterhin gehören hierzu Mono- oder Disaccharide, wie Glucose, Lactose, Saccharose oder Trehalose, Zuckeralkohole, wie Mannit, Sorbit, Xylit oder Glycerin, oder wasserlösliche Polymere, wie Dextrane oder Polyethylenglykole.

Das erfindungsgemäße Verfahren kann weiterhin zur Herstellung von Dosierungsformen angewandt werden, die für eine Vielzahl von Verabreichungstechniken einsetzbar sind, beispielsweise für die intravaskuläre, subarachnoidale, intraneurale und orale Verabreichung.

Weiterhin erlaubt das erfindungsgemäße Verfahren je nach diagnostischer Fragestellung eine Konzentrations- bzw. Volumenänderung des Kontrastmittels im Verlauf der Applikation.

So können automatisiert nacheinander verschiedene Konzentrationen bzw. Volumen in situ hergestellt und appliziert werden.

Auch ist es möglich, die ursprünglich vorgesehene Dosierungsform während der Untersuchung bezüglich Konzentraion oder Volumen zu ändern, falls die ursprünglich applizierte Dosis noch nicht das gewünschte diagnostische Ergebnis erbringen könnte.

Das Kontrastmittelkonzentrat liegt in einer fließfähigen Form vor, beispielsweise als Lösung, Dispersion oder Pulver. Im letztgenannten Fall kann das Lösungsmittel als Solvatationsmittel und/oder Dispersionsmedium wirken.

Die erfindungsgemäße Vorrichtung zur Durchführung des erfindungsgemäßen Verfahrens weist zunächst wenigstens einen Kontrastmittelkonzentratbehälter und wenigstens einen Verdünnungsmittelbehälter auf. Aus diesen Behältern wird jeweils eine vorbestimmte Menge an Fluid entnommen, wobei die Entnahme entweder gleichzeitig oder aufeinanderfolgend durchgeführt werden kann. Die jeweils entnommenen vorbestimmten Fluidmengen werden einer Mischzone oder einer Mischkammer zugeführt, in denen die beiden Fluide vermischt werden, wobei ein einheitliches Kontrastmedium in der einsetzbaren Dosierungsform erzielt wird. Der Konzentratbehälter und der Verdünnungsmittelbehälter sind jeweils über Leitungen mit der Mischzone verbunden. Die Förderung der Fluide kann entweder selbsttätig infolge der Schwerkrafteinwirkung oder durch aktive Pumpbeeinflussung mittels einer Pumpe, beispielsweise einer Vakuumpumpe oder einer peristaltischen Pumpe, erfolgen.

Sofern die Förderung unter Schwerkrafteinwirkung erfolgt, sind in den Zuflußleitungen zur Mischzone jeweils Absperreinrichtungen, beispielsweise Ventile, vorgesehen, die in einer vorbestimmten Weise von einer Kontrolleinheit aktiviert werden. Dabei bestimmen die Menge des in dem jeweiligen Behälter vorliegenden Fluids und der Leitungsquerschnitt sowie die Leitungslänge die zufließende Fluidmenge sowie die Zuflußgeschwindigkeit des Fluids.

Wenn jedoch Pumpen eingesetzt werden, so wirkt eine peristaltische Pumpe im nichtaktivierten Zustand als Absperreinheit, pumpt jedoch im aktivierten Zustand das Fluid aus dem jeweiligen Behälter in die Mischzone. Falls das Fluid mit Hilfe von Vakuum in die Mischzone gepumpt wird, sind wiederum Absperrorgane in den Zuflußleitungen angeordnet, die zusammen mit der Vakuumeinheit aktiviert werden.

Die Zuflußleitungen können entweder direkt mit der Mischkammer verbunden sein oder aber auch vor der Mischkammer sich vereinigen, wobei von dem Vereinigungspunkt eine einzige Leitung zur Mischkammer abgeht, so daß eine Y-Verbindung gebildet wird.

Die vorstehend beschriebenen Pump- und Absperreinrichtungen arbeiten üblicherweise nicht ausreichend genau, so daß zur genauen Bestimmung der Menge und der Konzentration der Dosierungsform vorteilhafterweise eine Einheit zur Bestimmung der Fluidmengen vorgesehen ist, mit der die aus dem jeweiligen Behälter entfernte Fluidmenge bestimmt werden kann. Zu solchen Einheiten gehören beispielsweise Durchflußsensoren in den Zuflußleitungen, mit denen die pro Zeiteinheit durchgeflossene Fluidmenge bestimmt werden kann. Andererseits können auch Waagen eingesetzt werden, die entweder die Zunahme der Fluidmengen in der Mischkammer oder aber die jeweilige Abnahme der Fluidmengen in dem jeweiligen Behälter bestimmen. Schließlich können auch volumetrisch arbeitende Einheiten an der Mischkammer bzw. dem Ausgangsbehälter angeordnet sein, mit denen das zufließende Volumen bzw. die abgeflossenen Volumina ermittelt werden können.

In jedem Fall können mit diesen Sensoren exakt die ab- bzw. zugeflossenen Fluidmengen oder Fluidvolumina in Abhängigkeit von der Aktivierungszeit der Absperrorgane bzw. Pumpen bestimmt werden. Diese Sensoren sind mit der Kontrolleinheit elektrisch verknüpft und senden entsprechende Signale als jeweiligen Istwert an die Kontrolleinheit.

Die Kontrolleinheit selbst errechnet aus den übermittelten Signalen die jeweiligen Mengen, Volumina und/oder Konzentrationen der geförderten Massen bzw. der aktuell in der Mischkammer befindlichen Mischung. Bekanntlich lassen sich über die spezifische(n) Dichte(n) der eingesetzten Kontrastmittelverbindung(en) gewünschte Konzentrationen der Darreichungsform sicher steuern.

Bei jodierten Röntgenkontrastmedien tritt typischerweise eine hohe Dichte auf. Hier kann die Dichte als einsetzbarer Indikator für die Konzentrationsbestimmung eingesetzt werden. In einem solchen Fall ist ein Dichtesensor innerhalb der Mischkammer vorgesehen. Andererseits kann auch die optische Drehung ein einsetzbarer Bestimmungsparameter sein. Selbstverständlich können u.a. auch volumetrische oder gravimetrische Bestimmungsparameter herangezogen werden.

Wie vorstehend festgestellt, dient das Ist-Signal für die Kontrolleinheit zur Steuerung der Pumpen bzw. der Absperrorgane in Abhängigkeit von einem zuvor eingestellten Sollwert, der manuell über eine Eingabeeinheit in die Kontrolleinheit eingegeben werden kann.

Die Kontrolleinheit sendet entsprechende Steuersignale zur Betätigung der Absperrorgane und Pumpen und empfängt die Sensorsignale, die die Fluidmengen betreffen, die aus den Behältern entnommen worden sind und/oder an die Mischkammer geliefert worden sind.

Weiterhin kann die Mischkammer über eine Abzugsleitung mit einem Mittel zur Verabreichung des Kontrastmediums, beispielsweise Injektionsspritzen, verbunden sein, die nacheinander mit dem Kontrastmittel in der gewünschten Dosierungsform gefüllt werden. Schließlich kann die Mischkammer alternativ mit einem oder mehreren Behältern zur Speicherung von einer oder mehreren Kartuschen für Druckinjektoren oder direkt an einen Patienten für eine Direktinfusion angeschlossen sein.

Die von der Mischkammer abgehende Abzugsleitung ist dabei ebenfalls mit einem Absperrorgan versehen, das während des Mischvorgangs geschlossen ist und erst zu Abfüllzwecken geöffnet wird.

Gemäß einer weiteren vorteilhaften Ausführungsform ist die Zuleitungsanordnung mit einem zusätzlichen Behälter verbunden, der eine Reinigungs- und Spülflüssigkeit aufweist. Hierdurch können die Leitungen, Absperrorgane, Pumpen, die Mischkammer, die Sammelkammer, die Aufnahmebehälter und dergl. gespült werden. Als Flüssigkeit kann hierfür jedes geeignete Reinigungsmedium in Frage kommen, beispielsweise steriles oder unsteriles Wasser mit oder ohne Additive. Zusätzlich kann das Verdünnungsmittel selbst als Spülfluid verwendet werden.

Die erfindungsgemäße Anordnung kann weiterhin mit einer Sammelkammer verbunden sein, in der das während der Reinigungsstufe eingesetzte Fluid gesammelt werden kann.

Die Mischkammer kann mit leeren Behälter, die beispielsweise durch ein Septum verschlossen sind, verbunden werden. In solchen sterilen Behältern wird das Kontrastmittel in seiner Dosierungsform gehalten. Es kommen hierbei sterile Infusions- oder Injektionsflaschen aus Plastik oder Glas in Frage, wie auch Infusionsbeutel.

Gemäß einer weiteren Ausführungsform kann die erfindungsgemäße Anordnung direkt an einen Extruder angeschlossen sein, mit dem entsprechende Plastikbehälter hergestellt werden. Schließlich kann die Anordnung dazu verwendet werden, daß derartige leere Behälter oder eine Injektionsspritze direkt gefüllt werden oder aber es kann eine Direktinfusion am Patienten durchgeführt werden.

Gemäß einer weiteren vorteilhaften Ausführungsform ist die erfindungsgemäße Anordnung mit einer Sterilisierungseinheit verbunden. Eine solche Sterilisierungseinheit kann beispielsweise einen Dampferzeuger aufweisen, der über eine Verbindungsleitung mit der gesamten Leitungsanordnung und dem Absperrorgan, Pumpen und den Behältern etc. verbunden ist. Andererseits kann als Sterilisierungseinheit auch ein flüssiges Desinfektionsmittel, beispielsweise Peressigsäure, eingesetzt werden, wobei nach dem Sterilisierungsvorgang darauf zu achten ist, daß etwaige Desinfektionsmittelreste mit Sterilwasser entfernt werden. Schließlich wird die Sterilisation der erfindungsgemäßen Anordnung weniger häufig durchgeführt als das Spülen.

Der Kontrastmittelkonzentratbehälter kann jede gewünschte Größe aufweisen, beispielsweise 0,1-100 l, insbesondere etwa 1 l, 5 l, 10 l, 50 l udgl. Die Behälter können aus jedem geeigneten Material gefertigt sein, das zur Lagerung des konzentrierten pharmazeutisch akzeptierbaren Kontrastmediums geeignet ist und vorteilhafterweise sterilisiert werden kann, beispielsweise hitzestabiles Plastik, Glas oder Metall. Diese Behälter können entweder wiederverwendbar oder aber auch nur für den einmaligen Gebrauch geeignet sein.

Vorteilhafterweise wird die sterilisierte Vorrichtung mit Hilfe von in die Leitungsabschnitte eingeschaltete Sterilfilter im sterilisiertem Zustand gehalten. Desweiteren kann der Mischeffekt in der Mischzone oder Mischkammer dadurch verbessert werden, daß eine Rühreinrichtung in der Mischzone oder Mischkammer vorgesehen ist.

Das Kontrastmedium liegt innerhalb des Behälters in hoher Konzentration vor. Als konzentrierte Lösung und/oder Dispersion wird eine Lösung mit einer Konzentration angesehen, die entweder gleich oder höher ist als diejenige von handelsüblichen Dosierungsformen des Kontrastmittels. Für den Beispielsfall eines jodierten Röntgenkontrastmittels liegt die Konzentration des Kontrastmittels im Vorlagebehälter bei einer Menge von etwa 350 - 450 mg J/ml. Bei NMR-Kontrastmitteln, beispielsweise bei Gadopentetatdimeglumine (Dimeglumine Gd-DTPA) liegt die handelsübliche Konzentration üblicherweise bei etwa 0,5 mol/l. Demzufolge weisen konzentrierte Lösungen oder Dispersionen des NMR-Kontrastmittels einen gleichen oder höheren Konzentrationsgehalt auf, für gelöste Konzentrate beispielsweise größer gleich 0,5 mol/l, insbesondere einen Gehalt von 0,5-4 mol/l.

Mit dem erfindungsgemäßen Verfahren wird zwar vorzugsweise eine einsetzbare Dosierungsform aus einem konzentrierten Kontrastmedium hergestellt, es ist jedoch möglich, auch das erfindungsgemäße Verfahren zur Senkung der Konzentration von handelsüblichen Dosierungsformen von Kontrastmedien einzusetzen.

Gemäß einer weiteren Ausführungsform der Erfindung wird ein Set zur Verfügung gestellt, das in Verbindung mit dem vorstehend beschriebenen System zur Formulierung von einsetzbaren Dosierungsformen von Kontrastmedien eingesetzt werden kann. Ein solches Set weist einen ersten Behälter mit einem bestimmten Kontrastmedium und einen zweiten Behälter mit einem bestimmten geeigneten Verdünnungsmittel auf. Diese Behälter des Sets haben vorzugsweise Mittel zur Verbindung mit der erfindungsgemäßen Vorrichtung zur Überführung von vorbestimmten Fluidmengen von jedem der Behälter zur Mischkammer. Ein Set kann beispielsweise den ersten Behälter mit 0,1-100 Liter eines konzentrierten Röntgenkontrastmittels mit einer Konzentration von 350-450 mg J/ml und einen zweiten Behälter mit 1-100 Liter Verdünnungsmittel aufweisen, das für die Formulierung eines Röntgenkontrastmittels in pharmazeutisch akzeptierbaren Formen geeignet ist. Andererseits kann das Set auch einen ersten Behälter mit 100 ml bis 100 Liter eines NMR-Kontrastmittels mit einer Konzentration von 0,5-4 mol pro Liter enthalten.

Die Erfindung wird anhand eines Ausführungsbeispiels erläutert.

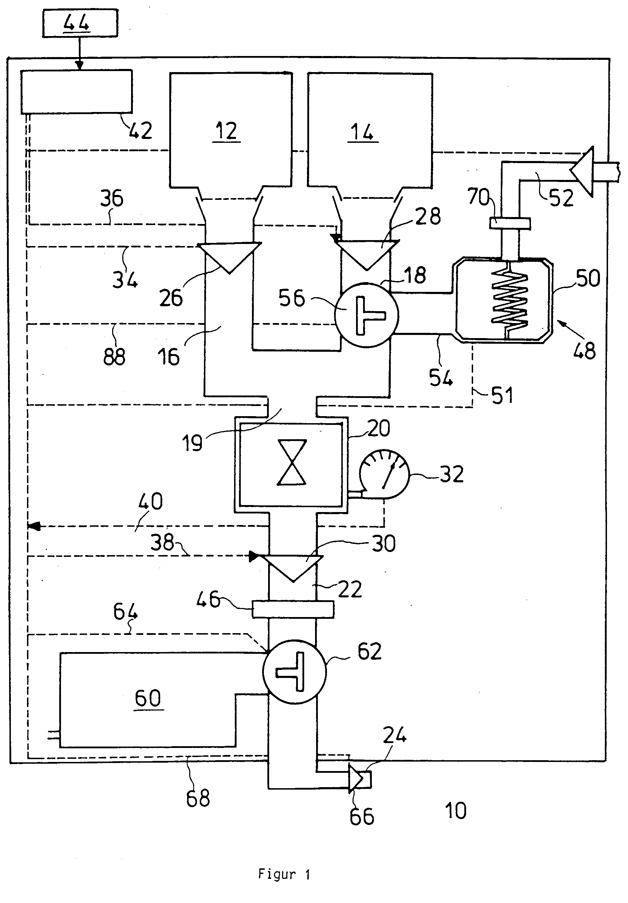

Figur 1 zeigt eine prinzipielle Skizze der erfindungsgemäßen Mischanordnung.

Die in Figur 1 gezeigte Vorrichtung 10 weist einen Behälter 12 für ein Kontrastmittelkonzentrat und einen Behälter 14 für ein Lösungs- oder Verdünnungsmittel auf. Die beiden Behälter 12 und 14 sind über Zuflußleitungen 16 und 18 mit einer Mischkammer 20 verbunden. In Beispiel sind die beiden Leitungen 16 und 18 stromauf der Mischkammer 20 zu einer einzigen Leitung 19 zusammengeführt, die dann in die Mischkammer 20 mündet. Eine solche Verbindung ist jedoch nicht notwendig. Dementsprechend können auch die beiden Leitungen 16 und 18 direkt mit der Mischkammer 20 verbunden sein.

Von der Mischkammer 20 geht eine Abzugsleitung 22 ab, deren Ende 24 mit nicht gezeigten Aufnahmebehältern (Flaschen, Beuteln oder Spritzenanordnungen) verbunden werden kann.

In die erste Zuflußleitung 16 ist ein erstes Dosierorgan 26 und in die zweite Zuflußleitung 18 ein zweites Dosierorgan 28 eingeschaltet. Desweiteren ist in die Abflußleitung 22 ein drittes Dosierorgan 30 eingeschaltet.

Die Mischkammer 20 ist mit einer Wägeeinrichtung 32 verbunden, die den jeweils aktuellen Gewichtszustand der Mischkammer 20 ermitteln kann.

Die Dosierorgane 26, 28 und 30 sowie die Wägeeinrichtung 32 sind über Signalleitungen 36-40 mit einer Kontrolleinheit 42 verbunden. Die Kontrolleinheit 42 weist weiterhin eine Eingabeeinheit 44 auf, mit der bestimmte Daten in die Kontrolleinheit 42 eingegeben werden können.

Gemäß einer vorteilhaften Ausführungsform ist stromab des dritten Dosierorgans 30 in der Abflußleitung 22 ein Sterilfilter 46 vorgesehen.

Desweiteren ist vorteilhafterweise die Vorrichtung 10 mit einer Sterilisierungseinheit 48 versehen, die beispielsweise ein Dampfgenerator 50 aufweist, der über eine Zuflußleitung 52 mit einer nicht gezeigten Frischwasserquelle verbunden ist. Dieser Dampfgenerator 50 ist über eine Abzugsleitung 54 mit den Zuflußleitungen 16, 18 verbunden, wobei am Verbindungspunkt eine Absperranordung 56 in Form eines Dreiwegeventils eingeschaltet ist. Diese Absperranordnung 56 ist mittels einer Signalleitung 58 ebenfalls mit der Kontrolleinheit 42 verbunden.

Stromab der Mischkammer 20 geht von der Abzugsleitung 22 eine Sammelkammer 60 ab, die zur Aufnahme von Spülmittellösungen und dergleichen vorgesehen ist. Diese Sammelkammer 60 ist ebenfalls über eine Absperranordnung 62 in Form eines Dreiwegeventils mit der Abzugsleitung 22 verbunden.

Die Aktivierung der Absperreinrichtung 62 erfolgt dabei über eine Signalleitung 64 mittels der Kontrolleinheit 42.

Weiterhin kann am Ende der Abzugsleitung 22 ein Ablaßventil 66 vorgesehen sein, das ebenfalls über eine Signalleitung 68 mit der Kontrolleinheit 42 verbunden ist. Schließlich ist der Dampfgenerator 50 über eine Signalleitung 51 mit der Kontrolleinheit 42 verbunden.

Die in Figur 1 gezeigte Vorrichtung 10 kann in den beiden Behältern 12 und 14 die vorstehend erwähnten Konzentrate und Verdünnungsmittel aufweisen. Dabei ist die Vorrichtung 10 nicht auf die beiden Behälter 12 und 14 beschränkt, sondern sie kann eine Vielzahl dieser Behälter aufweisen, die jeweils über getrennte Zuleitungen, in die jeweils entsprechende Absperr- oder Dosierorgane eingeschaltet sind, mit der Mischkammer verbunden sind.

Die in den Zugangsleitungen 16 und 18 und in der Abzugsleitung 22 eingesetzen Dosierorgane 26, 28 und 30 arbeiten infolge des Schwerkraftprinzips, d.h. im Aktivzustand sind die Dosierorgane 26,28 und 30 geöffnet und lassen im Öffnungszeitraum die jeweils stromauf befindlichen Fluide durch. Insofern wirken sie als Absperreinrichtungen. Sie können daher gleichermaßen durch andere Dosierungseinrichtungen ersetzt werden, beispielsweise peristaltische Pumpen, die im nichtaktiviertem Zustand als Absperrorgane wirken und im aktiviertem Zustand das jeweils stromauf befindliche Fluid fördern können.

Die in Figur 1 gezeigte Vorrichtung 10 wird folgendermaßen betrieben:

Das im Behälter 10 vorliegende Kontrastmedium weist - wie eingangs beschrieben - eine bestimmte Zusammensetzung und Konzentration auf. Sowohl das eingesetzte Kontrastmittel als auch dessen Konzentratkonzentration können über die Eingabeeinheit 44 in die Kontrolleinheit 42 eingegeben werden. Um ein fertiges einsetzbares Kontrastmittel in der Mischkammer 20 herzustellen, muß der Bediener über die Eingabeeinheit 44 die Menge des zu mischenden Kontrastmittels eingeben, woraufhin die Kontrolleinheit aus dem vorgegebenen Kontrastmittelkonzentrat und dem Verdünnungsmittel jeweils die aus den Behältern 12 und 14 in die Mischkammer zu fördernden Mengen errechnet. Nacheinander werden dann die Dosierorgane 26 und 28 geöffnet, wobei die jeweiligen Fluide aus dem Behälter 12 bzw. 14 in die Mischkammer 20 fließen. Der Inhalt der Mischkammer 20 wird dabei von der Waage 32 überwacht, die nach Erreichen der ersten von der Kontrolleinheit 40 ermittelten Menge, beispielsweise der Konzentratmenge, das Dosierorgan 26 schließt und das Dosierorgan 28 aktiviert. Nach Erreichen der zweiten Sollmenge in der Mischkammer 20 wird das zweite Dosierorgan 28 desaktiviert.

Anschließend wird das dritte Dosierorgan 30 und gegebenenfalls das Auslaßventil 66 aktiviert, um entweder einen Teil oder den gesamten Inhalt an Kontrastmittel, das in der Mischkammer 20 enthalten ist, zum Auslaß 24 der Abzugsleitung 22 zu befördern ist. Demzufolge kann also das dritte Dosierorgan 30 nicht nur zur Herstellung einer Zubereitung, sondern vielmehr auch von mehreren Zubereitungen aus der Mischkammer 20 dienen.

Da stromab des Dosierorgans 30 ein Sterilfilter 46 vorgesehen ist, bleiben sämtliche Einheiten, die sich stromauf dieses Sterilfilters 46 befinden, im sterilisierten Zustand.

Nach Entleerung der Mischkammer 20 bzw. der Behälter 12 und 14 wird die gesamte Vorrichtung 10 mittels der Sterilisierungseinheit 48 sterilisiert, wobei sterilisierend wirkender Wasserdampf durch die gesamte Leitungsanordnung 16, 18, 22, die Mischkammer 20 und den Sterilfilter 46 bis zur Sammelkammer 60 geführt wird.

Hinzuzufügen ist, daß die Wägeeinheit 32 zur Bestimmung der in die Mischkammer 20 geflossenen Fluidmengen durch eine Volumenmeßeinheit ersetzt sein kann.

Schließlich kann in der Zufuhrleitung 52 für Frischwasser auch ein Pyrogenfilter 70 vorgesehen sein, das eventuell im zufließendem Wasser vorliegende Pyrogene wirksam zurückhält. Dieses Sterilwasser kann ebenso wie das Verdünnungsmittel im Behälter 14 zu Spülzwecken in einem getrennten Spülgang eingesetzt werden.

## Patentansprüche

1. Verfahren zur Herstellung eines diagnostischen Kontrastmediums, bei dem man eine oder mehrere Kontrastmittelverbindungen mit einem wäßrigen Lösungsmittel vermischt, dadurch gekennzeichnet, daß man unter im wesentlichen sterilen Bedingungen eine vorbestimmte Menge von mindestens einem entweder festen, rieselfähigen Kontrastmittelkonzentrat oder mindestens einem flüssigen Kontrastmittelkonzentrat in einer Menge von 350 - 450 mg J/ml bei Röntgenkontrastmitteln bzw. 0,5 - 4 mol Kontrastmittelverbindung/Liter bei Kernresonanzkontrastmitteln aus mindestens einem ersten Behälter durch eine erste Leitung einer Mischkammer und eine vorbestimmte Menge Wasser oder einer wäßrigen Lösung aus einem Zweiten Behälter durch eine Zweite Leitung der Mischkammer zuführt, dort die zugeführten Substanzen Zu einer physiologisch verträglichen Darreichungsform vermischt und das erhaltene Gemisch einer Ablaßstation zuführt.

2. Verfahren nach Anspruch 1, dadurch gekennzeichnet, daß die Konzentrate in Flüssigkeits-, Dispersions- oder Pulverform vorliegen.

3. Verfahren nach Anspruch 1 oder 2, dadurch gekennzeichnet, daß man als Verbindungen für die Herstellung des Kontrastmittels Iotrolan, Iopromid, Iohexol, Iosimid, Metrizamid, Salze von Amidoessigsäure, Iotroxinsäure, Iopamidol, 5-Hydroxyacetamido-2,4,6- triiodo-isophthalsäure-(2,3-dihydroxy-N-methylpropyl)-(2-hydroxyethyl) -diamid, 3-Carbamoyl-5-[N-(2- hydroxyethyl)-acetamido]-2,4,6-triiodo-benzoesäure[(1RS,2SR) - 2,3-dihydroxy-1-hydroxymethylpropyl]amid, Dispersionen von Iodipamidethylester, Gadolinium DTPA, Gadolinium DOTA, den Gadoliniumkomplex von 10[1-Hydroxymethyl-2,3-dihydroxypropyl]-1,4,7-tris-[(carboxymethyl)-1,4,7,10-tetraazacyclodecan], Eisen- oder Manganporphyrinchelate, stabile Magnetitdispersionen, Dispersionen von Galaktosemikropartikeln mit oder ohne Additive in Wasser, eine Galaktoselösung oder Dispersionen von Mikrokugeln von eingeschlossener Luft, insbesondere Cyanacrylate oder Albuminmikrokugeln einsetzt.

4. Verfahren nach Anspruch 1-3, dadurch gekennzeichnet, daß man Wasser einsetzt, welches Puffersubstanzen und/oder isotonisch wirkende Additive und/oder Komplexierungsmittel und/oder Kontrastmittel in einer Konzentration von 0-0,5 mol/l bzw. 0-350 mg J/l bzw. 0-100 mg Partikel/ml und/oder medizinisch wirksame Substanzen enthält.

5. Vorrichtung zur Herstellung eines Kontrastmittels, insbesondere nach Anspruch 1, mit wenigstens einem Konzentratbehälter, wenigstens einem Verdünnungsmittelbehälter, Zuflußleitungen, die jeweils von dem Konzentratbehälter und dem Verdünnungsmittelbehälter abgehen, einer Abzugsleitung, die mit den Zuflußleitungen in Strömungsverbindung ist, Dosierorganen in den Zuflußleitungen, die im aktivierten Zustand vorbestimmte Mengen Konzentrat und Verdünnungsmittel dosieren, Mittel zur Bestimmung der Mengen/Volumina an dosiertem Konzentrat und Verdünnungsmittel und mit einer Kontrolleinheit, die die Dosierorgane in vorbestimmter Weise aktiviert, dadurch gekennzeichnet, daß der Konzentratbehälter (12) ein Kontrastmittelkonzentrat aufweist, eine Mischkammer (20) zwischen den Zuflußleitungen (16, 18) und der Abzugsleitung (22) vorgesehen ist und in der Abzugsleitung (22) eine dritte Dosiereinrichtung (30) angeordnet ist, die im aktivierten Zustand fertiges Kontrastmittel zum Ende (24) der Abzugsleitung (22) abgibt.

6. Vorrichtung nach Anspruch 5, dadurch gekennzeichnet, daß die Mischkammer (20) mit einer Wägeeinrichtung (32) verbunden ist, deren Signal die Kontrolleinheit (42) als Sollwert sowohl im Zufluß- als auch im Ablußbetrieb zur Steuerung der Absperrorgane in den Zuflußleitungen (16, 18) und in der Abzugsleitung (22) empfängt.

7. Vorrichtung nach Anspruch 5, dadurch gekennzeichnet, daß das Ende (24) der Abzugsleitung (22) mit einem Behälter oder einer Spritze zur Aufnahme des fertigen Kontrastmittels oder direkt mit einem Patienten verbindbar ist.

8. Set zur Herstellung eines Kontrastmittels, enthaltend ein Kontrastmittelkonzentrat in einer Menge von 350 - 450 mg J/ml bei Röntgenkontrastmitteln oder 0,5 - 4 mol Kontrastmittelverbindung/Liter bei Kernresonanzkontrastmitteln in einem ersten Behälter mit einem Inhalt von 0,1 - 100 l und ein wäßriges Verdünnungsmittel in einem zweiten Behälter mit einem Inhalt von 1 - 100 l zur Verwendung in der Vorrichtung gemäß Anspruch 5.

9. Set nach Anspruch 8, dadurch gekennzeichnet, daß das Konzentrat als Verbindung Iotrolan, Iopromid, Iohexol, Iosimid, Metrizamid, Salze von Amidoessigsäure, Iotroxinsäure, Iopamidol, 5-Hydroxyacetamido-2,4,6- triiodo-isophthalsäure-(2,3-dihydroxy-N-methylpropyl)-(2-hydroxyethyl) -diamid, 3-Carbamoyl-5-[N-(2- hydroxyethyl)acetamido]-2,4,6-triiodo-benzoesäure-[(1RS,2SR)-2,3-dihydroxy-1-hydroxymethylpropyl]amid, Dispersionen von Iodipamidethylester, Gadolinium DTPA, Gadolinium DOTA, den Gadoliniumkomplex von 10[1-Hydroxymethyl-2,3-dihydroxypropyl]1,4,7-tris-[(carboxymethyl)-1,4,7,10- tetraazacyclodecan], Eisen- oder Manganporphyrinchelate, stabile Magnetitdispersionen, Dispersionen von Galaktosemikropartikeln mit oder ohne Additiv in Wasser oder einem anderen wässrigen Diluent, eine Galaktoselösung oder Dispersionen von Mikrokugeln von eingeschlossener Luft, insbesondere aus Cyanacrylaten oder Albuminmikrokugeln bei Kernresonanzkontrastmitteln enthält.

## Claims

1. Process for the preparation of a diagnostic contrast medium, wherein one or more contrast agent compounds are mixed together with an aqueous solvent, characterised in that, under substantially sterile conditions, a pre-determined amount of either at least one solid, pourable contrast agent concentrate or at least one liquid contrast agent concentrate, in an amount of from 350 to 450 mg of iodine/ml in the case of X-ray contrast agents or from 0.5 to 4 mol of contrast agent compound per litre in the case of nuclear resonance contrast agents, is supplied from at least a first container through a first line to a mixing chamber, and a pre-determined amount of water or of an aqueous solution is supplied from a second container through a second line to the mixing chamber, the supplied substances are mixed there to form a physiologically tolerable presentation form and the resulting mixture is fed to an outlet location.

2. Process according to claim 1, characterised in that the concentrates are in the form of liquids, dispersions or powders.

3. Process according to claim 1 or 2, characterised in that there are used as compounds for the preparation of the contrast agent iotrolan, iopromide, iohexol, iosimide, metrizamide, salts of amidoacetic acid, iotroxic acid, iopamidol, 5-hydroxyacetamido-2,4,6-triiodo-isophthalic acid (2,3-dihydroxy-N-methylpropyl)-(2-hydroxyethyl)-diamide, 3-carbamoyl-5-[N-(2-hydroxyethyl)-acetamido]-2,4,6-triiodobenzoic acid [(1RS,2SR)-2,3-dihydroxy-1-hydroxymethylpropyl]amide, dispersions of iodipamide ethyl ester, gadolinium DTPA, gadolinium DOTA, the gadolinium complex of 10-[1-hydroxymethyl-2,3-dihydroxypropyl]-1,4,7-tris[(carboxymethyl)-1,4,7,10-tetraazacyclododecane], iron or manganese porphyrin chelates, stable magnetite dispersions, dispersions of galactose microparticles with or without additives in water, a galactose solution or dispersions of microspheres of encapsulated air, especially cyanoacrylates or albumin microspheres.

4. Process according to claim 1-3, characterised in that water that comprises buffer substances and/or isotonically active additives and/or complexing agents and/or contrast agents in a concentration of from 0 to 0.5 mol/litre or from 0 to 350 mg of iodine/litre or from 0 to 100 mg of particles/ml, and/or medically active substances is used.

5. Device for the preparation of a contrast agent, especially according to claim 1, having at least one concentrate container, at least one diluent container, supply lines leading in each case from the concentrate container and the diluent container, a discharge line in flow communication with the supply lines, metering devices in the supply lines, which metering devices in the activated state meter pre-determined amounts of concentrate and diluent, means for determining the amounts/volumes of metered concentrate and diluent, and a control unit, which activates the metering devices in a pre-determined manner, characterised in that the concentrate container (12) contains a contrast agent concentrate, a mixing chamber (20) is provided between the supply lines (16, 18) and the discharge line (22) and there is arranged in the discharge line (22) a third metering device (30), which in the activated state delivers finished contrast agent at the end (24) of the discharge line (22).

6. Device according to claim 5, characterised in that the mixing chamber (20) is connected to a weighing device (32), the signal of which is received as target value by the control unit (42) during both supply and discharge operations for controlling the shut-off devices in the supply lines (16, 18) and in the discharge line (22).

7. Device according to claim 5, characterised in that the end (24) of the discharge line (22) is arranged to be connected to a container or to a syringe for receiving the finished contrast agent or directly to a patient.

8. Kit for the preparation of a contrast agent, for use in the device according to claim 5, comprising in a first container having a capacity of from 0.1 to 100 litres a contrast agent concentrate in an amount of from 350 to 450 mg of iodine/ml in the case of X-ray contrast agents or from 0.5 to 4 mol of contrast agent compound/litre in the case of nuclear resonance contrast agents and in a second container having a capacity of from 1 to 100 litres an aqueous diluent.

9. Kit according to claim 8, characterised in that the concentrate comprises as compound iotrolan, iopromide, iohexol, iosimide, metrizamide, salts of amidoacetic acid, iotroxic acid, iopamidol, 5-hydroxyacetamido-2,4,6-triiodo-isophthalic acid (2,3-dihydroxy-N-methylpropyl)-(2-hydroxyethyl)-diamide, 3-carbamoyl-5-[N-(2-hydroxyethyl)-acetamido]-2,4,6-triiodobenzoic acid [(1RS,2SR)-2,3-dihydroxy-1-hydroxymethylpropyl]amide, dispersions of iodipamide ethyl ester, gadolinium DTPA, gadolinium DOTA, the gadolinium complex of 10-[1-hydroxymethyl-2,3-dihydroxypropyl]-1,4,7-tris[(carboxymethyl)-1,4,7,10-tetraazacyclododecane], iron or manganese porphyrin chelates, stable magnetite dispersions, dispersions of galactose microparticles with or without additives in water or another aqueous diluent, a galactose solution or dispersions of microspheres of encapsulated air, especially of cyanoacrylates or albumin microspheres in the case of nuclear resonance contrast agents.

## Revendications

1. Procédé pour la préparation d'un produit de contraste diagnostique, dans lequel on mélange un ou plusieurs composés de produit de contraste avec une solution aqueuse, caractérisé en ce que l'on apporte, sous conditions essentiellement stériles, une quantité fixée auparavant d'au moins un concentré de produit de contraste soit solide fluide, soit au moins un concentré de produit de contraste liquide, dans une quantité de 350 à 450 mg I/ml pour des produits de contraste pour la radiographie aux rayons X ou de 0,5 à 4 moles de produit de contraste/litre pour les produits de contraste par la RMN, à partir d'au moins un premier récipient par une première conduite dans une chambre de mélange et une quantité d'eau ou d'une solution aqueuse fixée auparavant à partir d'un deuxième récipient par l'intermédiaire d'une deuxième conduite à la chambre de mélange, dans laquelle chambre de mélange sont mélangées les substances apportées pour obtenir une forme d'administration physiologiquement tolérée, et on amène le mélange obtenu à un poste de décharge.

2. Procédé selon la revendication 1, caractérisé en ce que les concentrés se présentent sous forme de liquides, de dispersions ou de poudres.

3. Procédé selon la revendication 1 ou 2, caractérisé en ce que l'on utilise pour la préparation du produit de contraste l'iotrolane, l'iopromide, l'iohexol, l'iosimide, le metrizamide, des sels d'acide amidoacétique, l'acide iotroxinique, l'iopamidol, le (2,3-dihydroxy-N-méthyl-propyl)-(2-hydroxyéthyl)-diamide d'acide 5-hydroxy-acétamido-2,4,6-triiodoisophtalique, le [(1RS,2SR)-2,3-dihydroxy-1-hydroxy-méthylpropyl]amide d'acide 3-carbamoyl-5-[N-(2-hydroxy-éthyl)-acétamido]-2,4,6-tri-iodobenzoique et des dispersions d'esters de iodipamide, le complexe de Gadolinium DTPA, le gadolinium DOTA, le complexe de gadolinium de 10-[1-hydroxyméthyl-2,3-dihydroxypropyl]-1,4,7-tris-[(carboxyméthyl)-1,4,7,10-tétraazacyclodécane], des chélates de porphyrine de fer ou porphyrine de manganèse, des dispersions de magnétite stables, des dispersions aqueuses de microparticules de galactose avec ou sans additif, une solution de galactose ou des dispersions de microbilles avec de l'air encapsulé, plus particulièrement en cyanoacrylate ou en albumine.

4. Procédé selon la revendication 1 à 3, caractérisé en ce que l'on utilise de l'eau, laquelle eau peut contenir des substances tampons et/ou des additifs à effet isotonique et/ou des agents complexants et/ou des produits de contraste dans une concentration de 0 à 0,5 mole/litre, respectivement de 0 à 350 mg I/l, respectivement de 0 à 100 mg de particules/ml et/ou des substances médicinales actives.

5. Dispositif pour la préparation d'un produit de contraste, plus particulièrement selon la revendication 1, comportant au moins un récipient de concentré, au moins un récipient de diluant, des conduites d'alimentation lesquelles conduites d'alimentation partent à chaque fois depuis le récipient de concentré et le récipient de diluant, d'une conduite de soutirage, laquelle est reliée par écoulement aux conduites d'alimentation, des organes de dosage dans les conduites d'alimentation, lesquels, à l'état activé, mesurent des quantités fixées de concentré et de diluant, des organes pour déterminer les quantités/volumes de concentré et de diluant dosés et reliés à l'unité de contrôle qui active de façon prédéterminée les organes de dosage, caractérisé en ce que le récipient de concentré (12) présente un concentré de produit de contraste, une chambre de mélange (20) étant prévue entre les conduites d'aliementation (16, 18) et la conduite de soutirage (22) et dans la conduite de soutirage (22) étant disposé un troisième dispositif de dosage (30), lequel à l'état activé débite le produit de contraste fini à l'extrémité (24) de la conduite de soutirage (22).

6. Dispositif selon la revendication 5, caractérisé en ce que la chambre de mélange (20) est raccordée à un dispositif de pesage (32), dont le signal est accepté par l'unité de contrôle (42) en tant que donnée fixée aussi bien à l'état d'alimentation qu'à l'état d'évacuation pour le pilotage des organes d'obturation dans les conduites d'alimentation (16, 18) et dans la conduite de soutirage (22).

7. Dispositif selon la revendication 5, caractérisé en ce que l'extrémité (24) de la conduite de soutirage (22) peut être raccordée à un récipient ou à une seringue d'injection pour recueillir le produit de contraste fini ou directement au patient.

8. Ensemble pour la préparation d'un produit de contraste contenant un concentré de produit de contraste dans une quantité de 350 à 450 I/ml pour les produits de contraste pour la radiographie aux rayons X, ou 0,5 à 4 moles de produit de contraste/litre pour les produits de contraste par la RMN, dans un premier récipient ayant un volume de 0,1 à 100 litres et un agent diluant aqueux dans un deuxième récipient ayant un volume de 1 à 100 litres pour l'utilisation dans le dispositif selon la revendication 5.

9. Ensemble selon la revendication 8, caractérisé en ce que le concentré contient en tant que composé l'iotrolane, l'iopromide, l'iohexol, l'iosimide, le metrizamide, des sels d'acide amidoacétique, l'acide iotroxinique, l'iopamidol, le (2,3-dihydroxy-N-méthylpropyl)-(2-hydroxyéthyl)-diamide d'acide 5-hydroxy-acétamido-2,4,6-triiodo-isophtalique, le [(1RS,2SR)-2,3-dihydroxy-1-hydroxy-méthylpropyl]amide d'acide 3-carbamoyl-5-[N-(2-hydroxyéthyl)-acétamido]-2,4,6-triiodo-benzoique et des dispersions d'ester d'éthyle d'iodipamide, le gadolinium DTPA, le gadolinium DOTA, le complexe de gadolinium de 10-[1-hydroxyméthyl-2,3-dihydroxypropyl]-1,4,7-tris-[(carboxyméthyl)-1,4,7,10-tétraazacyclo-décane], des chélates de porphyrine de fer ou de porphyrine de manganèse, des dispersions de magnétite stables, des dispersions aqueuses de microparticules de galactose avec ou sans additif ou dans un autre diluant aqueux, une solution de galactose ou des dispersions de microparticules avec de l'air encapsulé, plus particulièrement des microparticules en cyanoacrylate ou en albumine, dans le cas de produits de contraste par la RMN.
